# EUROPEAN PATENT SPECIFICATION

(11) **EP 0 712 443 B1**
(45) Date of publication and mention of the grant of the patent: **26.11.1997**
(21) Application number: 94924421.4
(22) Date of filing: 13.07.1994
(51) Int. Cl.: C12P 35/04, C12P 37/04, C12P 17/10, C07C 231/20

(54) **PROCESS FOR THE ENZYMATIC PREPARATION OF A BETA-LACTAM DERIVATIVE AND SEPARATION OF D-PHENYLGLYCINE AMIDE**
VERFAHREN ZUR ENZYMATISCHEN HERSTELLUNG VON BETA-LACTAM DERIVATEN UND ABTRENNUNG VON D-PHENYLGLYCINE AMID
PROCEDE DE PRODUCTION ENZYMATIQUE D'UN DERIVE DE BETALACTAME ET SEPARATION DE D-PHENYLGLYCINE AMIDE

(30) Priority: 19.07.1993 BE 9300750
(43) Date of publication of application: 22.05.1996
(73) Proprietor: DSM N.V., 6411 TE Heerlen (NL)
(72) Inventor: BOESTEN, Wilhelmus, Hubertus, Joseph, NL-6132 BJ Sittard (NL); MOODY, Harold, Monro, NL-6211 BZ Maastricht (NL)
(86) International application number: NL9400161
(87) International publication number: WO9503420

(56) References cited:
- EP-A- 0 442 584
- EP-A- 0 442 585
- WO-A-92/01061
- WO-A-92/12782
- US-A- 4 172 846

## Description

The invention relates to a process for the preparation of a β-lactam derivative, wherein in an enzymatic reaction a β-lactam nucleus is coupled to D-phenylglycine amide, and the enzyme, the solid phenylglycine and the β-lactam derivative are separated out.

The enzymatic coupling of a β-lactam nucleus and an acylation agent to form a β-lactam derivative is described in WO-A-9201061. In particular esters and amides of phenylglycine and p-hydroxyphenylglycine are described as acylation agents. The recovery and purification of the product is effected in a known way, for instance by crystallization. Although the product can be obtained with a good yield and high purity in this way, in practice it is difficult to separate from each other for the purpose of re-use the substances that remain behind in the reaction mixture, such as non-converted phenylglycine amide, non-converted β-lactam nucleus, formed phenylglycine in solution, β-lactam derivative remaining in solution after crystallization.

Since in the enzymatic coupling reaction phenylglycine amide is present in a large excess relative to the β-lactam nucleus - the optimum molar ratio of phenylglycine amide to β-lactam nucleus is often between 2 and 9 - in order to secure a high yield from the enzymatic reaction, it is notably important to recover the non-converted phenylglycine amide in order to obtain a commercially attractive process.

The invention now provides a process whereby more than 85% of the phenylglycine amide can be recovered in pure form in a simple way, even before the β-lactam derivative is separated out.

This is achieved according to the invention in that the mixture obtained after the enzymatic reaction, from which mixture at least the enzyme and the solid phenylglycine have been removed, is treated with a suitable aldehyde at a pH between 7.5 and 8.5, whereby the Schiff base of phenylglycine amide is formed, and the Schiff base of phenylglycine amide is separated out.

For surprisingly it has been found that in this way the Schiff base of the phenylglycine amide is formed, while the aldehyde does not form Schiff bases with the other compounds (amines), which means that the other compounds remain dissolved in the mixture. This is all the more surprising as it is generally known that when amines are contacted with an aldehyde, they react to form a Schiff base and then precipitate as such. The β-lactams which are present in the reaction mixture appear to remain virtually completely in solution, however, while the phenylglycine amide appears to have virtually completely reacted with the aldehyde added.

It should also be pointed out that US-A-4172846 describes a process for the separation of L-phenylglycine from D-phenylglycine amide via formation of a Schiff base. That publication teaches nothing about the possible selective separation of phenylglycine amide from the mixture which also contains β-lactams. Moreover, in the examples the separation is in all cases carried out at a pH of about 10.5, which pH values are prohibitive in the present process on account of the instability of the β-lactam derivatives at such a high pH. The pH values in the process according to the invention are between 7.5 and 8.5. At such pH values, part of the phenylglycine amide will be present in the form of salt in the reaction mixture, as a result of which the formation of Schiff base is hindered.

The mixture that is obtained after the enzyme and the solid phenylglycine have been removed from the reaction mixture obtained after the enzymatic coupling reaction, will in general contain the β-lactam derivative formed, D-phenylglycine amide, remaining β-lactam nucleus and D-phenylglycine formed in quantities, relative to the β-lactam derivative formed, of for instance 1-7 eq. of D-phenylglycine amide, 0.02-1 eq. of β-lactam nucleus, 0.1-2 eq. of D-phenylglycine. The invention also relates to the removal of phenylglycine amide from such a mixture.

The process according to the invention also offers the advantage that D-phenylglycine amide can be separated out in a simple manner, for instance by filtration or extraction, and in a pure form. Preferably, the D-phenylglycine amide is separated out before the β-lactam derivative is recovered via crystallization. For this offers the additional advantage that the recovery of the β-lactam derivative as a product is simplified, since the solubility of the β-lactam derivative is higher when the D-phenylglycine amide is still present in the mixture. The β-lactam derivative can be recovered and purified in a known manner.

Within the framework of the invention, by β-lactam derivative is understood semisynthetic β-lactam obtained by acylation of a β-lactam nucleus with an activated form of D-phenylglycine as acylation agent. They are mostly used as antibiotics, such as Ampicilline, Cefalexine and Cefachlor. Known β-lactam nuclei are for instance 6-aminopenicillanic acid (6-APA), 7-aminocephalosporanic acid (7-ACA), 7-amino-3-chloro-3-cephem-4-carboxylate, 7-aminodesacetoxy-cephalosporanic acid (7-ACDA), etc.

The enzymatic coupling reaction can be carried out in a known manner, for instance as described in WO-A-92/01061.

The enzyme used in the process according to the invention can be any suitable enzyme which catalyzes the reaction. The enzyme may for be derived from known microorganisms such as Acetobaxter, Aeromonas, Aphanocladium, Athrobaxter, Bacillus Cephalosporium, Corynebacterium, Escherichia, Flavobacterium, Pseudomonas and Xanthomonas.

The D-phenylglycine amide that is used in the enzymatic reaction can be applied as free D-phenylglycine amide or as salt of an acid, for instance an acid with a pKₐ < 5, such as acetic acid, formic acid, sulphuric acid, hydrochloric acid or nitric acid.

The concentration of reactants is not critical and is preferably chosen such that upon completion of the enzymatic reaction all components, except for the D-phenylglycine formed, are still just in solution. Water can be used as solvent, but the use of an organic solvent is also possible. The temperature at which the enzymatic reaction is carried out is mostly between 0 and 40°C; the pH is mostly between 5 and 8.5.

Preferably, benzaldehyde is used in the formation of the Schiff base of D-phenylglycine amide.

Benzaldehyde is advantageous in that the separation of the Schiff base and the recovery of benzaldehyde are simple. Another advantage of benzaldehyde is that it is immiscible with water, so that as extraction agent it is preferred by far over other extraction agents, because the resulting Schiff base of the optically active phenylglycine amide dissolves in the benzaldehyde and the other components of the reaction mixture in the water phase.

The process according to the invention can also be carried out with other aldehydes, provided they meet the following requirements:
1. they should readily form water-immiscible Schiff bases of the D-phenylglycine amide;
2. the Schiff bases formed should readily decompose without decomposition of the aldehyde;
3. there should be a clear difference in the solubility in water and in organic solvents between the Schiff bases of the D-phenylglycine amide and the other components of the reaction mixture.

Benzaldehyde and substituted benzaldehydes are excellent examples of compounds that meet all these requirements very well.

By benzaldehyde are also understood substituted benzaldehydes, such as lower alkyl benzaldehyde, halogen benzaldehyde, nitrobenzyl aldehyde and lower alkoxybenzyl aldehyde. Lower alkyl or lower alkoxy means alkyl or alkoxy with 1-5 C-atoms.

The reaction with benzaldehyde with formation of Schiff base can be carried out at temperatures of 0-50°C, preferably 5-45°C. If for the formation of the Schiff base equimolar quantities of benzaldehyde, for instance 0.9-2, in particular 0.95-1.1 equivalents relative to the phenylglycine amide are applied without another solvent for the Schiff base of the amide, then a precipitate of the Schiff base of D-phenylglycine amide is obtained. The other components remain dissolved in the mother liquor. If an excess of benzaldehyde is used, the benzaldehyde will not only act as reaction agent but also as solvent, and two layers will be obtained. It is also possible to apply mixtures of benzaldehyde and other solvents, such as mixtures with toluene, chloroform, methylisobutyl ketone, tetrachloroethene, ethyl acetate and butyl acetate.

Recovery of the D-phenylglycine amide from the corresponding Schiff base can be simply effected through acidification with an equimolar quantity of strong acid, e.e. sulphuric acid (up to pH = 2-3), which results in decomposition of the Schiff base into the aldehyde and D-phenylglycine amide. The D-phenylglycine amide thus obtained can be supplied back to the enzymatic coupling step.

The invention will now be further elucidated by means of the following example.

### Example

To a 500 ml flask were successively supplied (corresponding to a reaction mixture obtained after enzymatic preparation of Cefalexine, followed by removal of the enzyme and solid D-phenylglycine): 0.56 g of 7-ADCA, 15.1 g of cefalexine.1 H₂O, 1.18 g of D-phenylglycine, 19.9 g of D-phenylglycine amide.½H₂SO₄, 185.4 g of H₂O, 8.1 g of H₂SO₄ (98%) and 19.8 g of 25% ammonia solution.

The pH of this solution (250.0 g) was 8.0.

At 20°C, 1 eq. (10.6 g) of benzaldehyde was added in 5 minutes with stirring. After 2 hours the resulting white solid substance was filtered off, washed with 3x20 ml H₂O and dried.

Yield of D-phenylglycine amide Schiff base: 20.8 g (> 99% pure); amounts of Cefalexine, phenylglycine and 7-ADCA each less than 0.5%.

## Claims

1. Process for the preparation of a β-lactam derivative, wherein in an enzymatic reaction a β-lactam nucleus is coupled to D-phenylglycine amide, and the enzyme, the solid D-phenylglycine and the β-lactam derivative are separated out, characterized in that from the mixture obtained after the enzymatic reaction at least the enzyme and the solid D-phenylglycine have been removed, the remaining mixture is treated with a suitable aldehyde at a pH between 7.5 and 8.5 whereby, the Schiff base of D-phenylglycine amide is separated out, and subsequently the β-lactamderivative is separated out.

2. Process for the preparation of a β-lactam derivative, wherein in an enzymatic reaction a β-lactam nucleus is coupled to D-phenylglycine amide, in which process the D-phenylglycine amide is recovered and the enzyme, the solid D-phenylglycine and the β-lactam derivative are separated out, characterized in that the mixture obtained after the enzymatic reaction, from which mixture at least the enzyme, the solid D-phenylglycine and the β-lactam derivative have been removed, is treated with a suitable aldehyde at a pH between 7.5 and 8.5 and the Schiff base of D-phenylglycine amide is separated out.

3. Process for the recovery of D-phenylglycine amide from a mixture containing a β-lactam derivative, D-phenylglycine amide, D-phenylglycine and a β-lactam nucleus, characterized in that the mixture is treated with a suitable aldehyde, whereby the Schiff base of D-phenylglycine amide is formed, and the Schiff base of D-phenylglycine amide is separated out.

4. Process according to claim 3, characterized in that the mixture contains, relative to the amount of β-lactam derivative, 1-7 equivalents of D-phenylglycine amide, 0.1-2 equivalents of D-phenylglycine and 0.02-1 equivalent of β-lactam nucleus.

5. Process according to any one of the claims 1-4, wherein the β-lactam nucleus is chosen from the group comprising 6-aminopenicillanic acid, 7-aminocephalosporanic acid, 7-amino-3-chloro-3-cephem-4-carboxylate and 7-aminodesacetoxy-cephalosporanic acid.

6. Process according to any one of the claims 1-5, wherein benzaldehyde is used as aldehyde.

7. Process according to any one of the claims 1-6, wherein the treatment with the aldehyde takes place at a temperature of 5-45°C.

8. Process according to any one of the claims 1-7, wherein 0.95-1.1 equivalents of benzaldehyde is applied, relative to the amount of D-phenylglycine amide.

## Patentansprüche

1. Verfahren zur Herstellung eines β-Lactam-Derivats, wobei in einer enzymatischen Reaktion ein β-Lactam-Kern mit D-Phenylglycinamid gekuppelt wird und das Enzym, das feste D-Phenylglycin und das β-Lactam-Derivat abgetrennt werden, dadurch gekennzeichnet, daß aus der nach der enzymatischen Reaktion erhaltenen Mischung zunächst zumindest das Enzym und das feste D-Phenylglycin entfernt worden sind, die übrige Mischung mit einem geeigneten Aldehyd bei einem pH-Wert von zwischen 7,5 und 8,5 behandelt wird, wodurch die Schiffsche Base des D-Phenylglycinamids abgetrennt wird und danach das β-Lactam-Derivat abgetrennt wird.

2. Verfahren zur Herstellung eines β-Lactam-Derivats, wobei in einer enzymatischen Reaktion ein β-Lactam-Kern mit D-Phenylglycinamid gekuppelt wird, bei welchem Verfahren das D-Phenylglycinamid zurückgewonnen wird und das Enzym, das feste D-Phenylglycin und das β-Lactam-Derivat abgetrennt werden, dadurch gekennzeichnet, daß die nach der enzymatischen Rekation erhaltene Mischung, aus welcher Mischung zumindest das Enzym, das feste D-Phenylglycin und das β-Lactam-Derivat entfernt worden sind, mit einem geeigneten Aldehyd bei einem pH-Wert zwischen 7,5 und 8,5 behandelt wird und die Schiffsche Base des D-Phenylglycinamids abgetrennt wird.

3. Verfahren zur Rückgewinnung eines D-Phenylglycinamids aus einer Mischung, welche ein β-Lactam-Derivat, D-Phenylglycinamid, D-Phenylglycin und einen β-Lactam-Kern enthält, dadurch gekennzeichnet, daß die Mischung mit einem geeigneten Aldehyd behandelt wird, wodurch die Schiffsche Base des D-Phenylglycinamids gebildet wird, und die Schiffsche Base des D-Phenylglycinamids abgetrennt wird.

4. Verfahren nach Anspruch 3, dadurch gekennzeichnet, daß die Mischung, bezogen auf die Menge an β-Lactam-Derivat, 1-7 Äquivalente D-Phenylglycinamid, 0,1-2 Äquivalente D-Phenylglycin und 0,02-1 Äquivalente β-Lactam-Kern enthält.

5. Verfahren nach einem der Ansprüche 1 bis 4, wobei der β-Lactam-Kern ausgewählt ist aus der Gruppe umfassend 6-Aminopenicillansäure, 7-Aminocephalosporansäure, 7-Amino-3-chlor-3-cephem-4-carboxylat und 7-Aminodesacetoxy-cephalosporansäure.

6. Verfahren nach einem der Ansprüche 1 bis 5, wobei Benzaldehyd als Aldehyd verwendet wird.

7. Verfahren nach einem der Ansprüche 1 bis 6, wobei die Behandlung mit dem Aldehyd bei einer Temperatur von 5-45°C erfolgt.

8. Verfahren nach einem der Ansprüche 1-7, wobei 0,95-1,1 Äquivalente Benzaldehyd, bezogen auf die Menge des D-Phenylglycinamids, verwendet werden.

## Revendications

1. Procédé pour la préparation d'un dérivé de β-lactame, dans lequel, dans une réaction enzymatique, un noyau de β-lactame est couplé avec du D-phénylglycine amide, et l'enzyme, la D-phénylglycine solide et le dérivé de β-lactame sont séparés, caractérisé en ce que, à partir du mélange obtenu, après la réaction enzymatique, au moins l'enzyme et la D-phénylglycine solide ont été prélevés, le mélange restant est traité avec un aldéhyde approprié à un pH entre 7,5 et 8,5, de façon que la base de Schiff du D-phénylglycine amide soit séparée et que, par la suite, le dérivé de β-lactame soit séparé.

2. Procédé pour la préparation d'un dérivé de β-lactame, dans lequel, dans une réaction enzymatique, un noyau de β-lactame est couplé à du D-phénylglycine amide, procédé dans lequel le D-phénylglycine amide est récupéré tandis que l'enzyme, la D-phénylglycine solide et le dérivé de β-lactame sont séparés, caractérisé en ce que le mélange obtenu après la réaction enzymatique, au moins l'enzyme, la D-phénylglycine solide et le dérivé de β-lactame ayant été éliminé de ce mélange, est traité avec un aldéhyde approprié à un pH entre 7,5 et 8,5, et la base de Schiff du D-phénylglycine amide est séparée.

3. Procédé pour la récupération du D-phénylglycine amide à partir d'un mélange renfermant un dérivé de β-lactame, du D-phénylglycine amide, de la D-phénylglycine et un noyau de β-lactame, caractérisé en ce que le mélange est traité avec un aldéhyde approprié, de façon que la base de Schiff du D-phénylglycine amide soit formée, après quoi la base de Schiff du D-phénylglycine amide est séparée.

4. Procédé selon la revendication 3, caractérisé en ce que le mélange renferme, par rapport à la quantité de dérivés de β-lactame, 1-7 équivalents de D-phénylglycine amide, 0,1-2 équivalents de D-phénylglycine et 0,02-l équivalent de noyau de β-lactame.

5. Procédé selon l'une quelconque des revendications 1 à 4, dans lequel le noyau de β-lactame est choisi dans le groupe comportant l'acide 6-aminopénicillanique, l'acide 7-aminocéphalosporanique, le 7-amino-3-chloro-3-céphem-4-carboxylate et l'acide 7-aminodésacétoxy-céphalosporanique.

6. Procédé selon l'une quelconque des revendications 1 à 5, dans lequel le benzaldéhyde est utilisé comme aldéhyde.

7. Procédé selon l'une quelconque des revendications 1 à 6, dans lequel le traitement avec l'aldéhyde est mis en oeuvre à une température de 5 à 45°C.

8. Procédé selon l'une quelconque des revendications 1 à 7, dans lequel on utilise de 0,95 à 1,1 équivalents de benzaldéhyde, par rapport à la quantité de D-phénylglycine amide.
